# EUROPEAN PATENT APPLICATION

(11) **EP 1 632 563 A1**
(43) Date of publication of application: **08.03.2006**
(21) Application number: 04742080.7
(22) Date of filing: 09.06.2004
(51) Int. Cl.: C12N 5/06

(54) **CARTILAGE-DERIVED STEM CELLS AND APPLICATIONS THEREOF**

(30) Priority: 12.06.2003 ES 200301386
(71) Applicant: CONSEJO SUPERIOR DE INVESTIGACIONES CIENTIFICAS, 28006 Madrid (ES); Cellerix, S.L., 28760 Tres Cantos (Madrid) (ES)
(72) Inventor: BERNAD MIANA, Antonio, Centro Nac'l biotecnologia, 28049 Madrid (ES); GONZALEZ DE LA PENA, Manuel Angel, Genetrix, E-28760 Tres Cantos (ES); DE LA FUENTE GONZALEZ, Ricardo, 28049 Madrid (ES)
(74) Representative: Pons Arino, Angel
(86) International application number: PCT/ES2004/070041
(87) International publication number: WO 2004/111208

(57) **Abstract**

The invention relates to methods of isolating adult stem cells, to the cells thus isolated and to applications thereof. More specifically, the invention relates to isolated adult stem cells which are derived from dedifferentiated chondrocytes, which can be differentiated and which can give rise to a series of cell lineages, as well as to specific markers present in said cells, such as cell surface antigens. The cells provided by the present invention can be used, for example, in cell therapy and in the search for and development of novel medicaments.

## Description

The invention relates to methods of isolating adult stem cells, to the cells thus isolated and to applications thereof. More specifically, the invention relates to isolated adult stem cells which are derived from dedifferentiated chondrocytes, which can be differentiated and which can give rise to a series of cell lineages, as well as to specific markers present in said cells, such as cell surface antigens. The cells provided by the present invention can be used, for example, in cell therapy and in the search for and development of novel medicaments.

### BACKGROUND OF THE INVENTION

Organ and tissue transplants provide a series of promising treatments for diverse pathologies, thus converting regenerative theory into the central target of research in many fields of biomedicine. However, there are two important problems associated with organ and tissue transplants. The first and most serious of these is the scarcity of donors. Thus, for example, the United States has only 5% of the organs it needs for transplants (Evans et al., 1992).

Secondly, there is the problem of the potential incompatibility of the transplanted tissue with the immune system of the recipient. Said incompatibility means that the transplanted organ or tissue is recognised as a foreign element by the recipient's immune system, making it necessary to administer the transplant patient immune suppressant drugs for the rest of his/her life which takes a high toll, both physical and economical. One possible solution to the shortage of organ and tissue donors could be the use of animal organs or tissues, a process known as xenotransplant. However, with that approach the problem of rejection is even worse and contributes to serious risks of the transmission of animal pathogens to humans (Patience, et al., 1997; Wilson et al., 1998).

Currently, technological development in the field of stem cell research has led them to be considered a promising source of organs and tissues for those types of pathologies requiring organ or tissue transplants. Theoretically, the stem cells can undergo cellular division for self-maintenance during an unlimited period of time to originate phenotypically and genotypically identical cells. Furthermore, they have the capacity to differentiate between one or several cell types in the presence of certain signals or stimuli.

The generation of organs and cells from the stem cells of the patient or from immunocompatible heterologous cells so that the immune system of the recipient does not recognise them as foreign offers a series of associated advantages that solve the problems brought on by the scarcity of donors and the risk of rejection. The use of stem cells for organ and tissue regeneration constitutes a promising alternative therapy for diverse human pathologies including: chondral, bone and muscular lesions, neurodegenerative diseases, immunological rejection, cardiac disease and skin disorders (see US patents 5.811.094, 5.958.767, 6.238.960, 6.379.953, 6.497.875).

In addition to cellular therapy applications, stem cells have many other potential applications related to biomedical technologies that can help to facilitate biopharmaceutical research and development activities. One of these applications lies in the development of cellular models of human and animal disease that can help to substantially improve the celerity and efficacy of the process of searching for and developing new drugs. At this time, the methods most commonly used to measure the biological activity of a new compound before it goes into clinical trials consist of incomplete biochemical techniques or costly and inadequate animal models. Stem cells could be a potential source of virtually unlimited quantities of cells, both undifferentiated and differentiated, for conducting in *vitro tests* to search for and develop new therapeutic compounds (US patent 6.294.346) and to determine their activity, metabolism and toxicity. The development of such tests, particularly high-throughput screening (HTS), would reduce the time and money needed to develop compounds with therapeutic activity, eliminate, to a large extent, the need to use animals for experimentation and would also reduce the exposure of patients to the adverse effects of the compounds during clinical trials. In addition, the availability of different types of cells from various individuals would provide a better understanding of the effects of a potentially therapeutic compound on a specific individual, leading to the full development of the pharmacogenomic field, where the activity of a compound would be correlated with the individual's genetic structure. The stem cells and their differentiated progeny are also very valuable in the process of searching for and characterising new genes involved in a wide variety of biological processes including development, cellular differentiation and neoplastic processes (Phillips et al., 2000; Ramalho-Santos et al., 2002; Ivanova et al., 2002). Gene expression systems for use in combination with cell-based HTS systems have already been described (Jayawickreme and Kost, 1997).

Depending on the origin of the stem cells, we can differentiate between embryonic stem cells (ES cells) and adult stem cells. The ES cells come from the internal cellular mass of the blastocyte and their most relevant feature is the fact that they are pluripotential, which means that they can give rise to any adult tissue derived from the three embryonic layers (Evans and Kaufman, 1981; Thomson et al., 1998; US Patent 6.200.806). Adult stem cells are partially compromised cells present in adult tissue which can remain in the human body for decades although they become scarcer with the passage of time (Fuchs and Segre, 2002).

Despite the high pluripotentiality of ES stem cells, therapies based on the use of adult stem cells offer a series of advantages over those based on ES cells. First of all, it is complicated to control the culturing conditions of ES cells without inducing their differentiation (Thomson et al., 1998), which raises the economic cost and the work required to use these types of cells. Furthermore, ES cells must go through several intermediate stages before they become the specific cell type needed to treat a particular pathology, a process that is controlled by chemically complex compounds. Moreover, there is heated controversy in relation to ES cells due to the extended belief that human life begins with fertilisation, so that the informed consent signed by the donors does not eliminate the ethical stigma associated with the use of embryos in research. There are also problems related to the safety of the therapeutic use of ES cells due to the high probabilities that the undifferentiated stem cells from embryonic tissue will produce a type of tumour known as teratocarcinoma (Evans and Kaufman, 1981).

Finally, the cells derived from ES cells are usually rejected by the immunological system due to the fact that the immunological profile of such cells differs from that of the recipient. Although this problem could be addressed by using a process known as "therapeutic cloning", in which autologous ES cells can be obtained by transferring the nucleus of a somatic cell from a patient to the ovocyte of a female donor, this technique has not yet been developed in humans and poses serious ethical and legal problems (human cloning is illegal in many countries). Another solution could be the generation of "universal" cellular lines with generalised immune compatibility, but there is no technology at this time that allows the obtention of such cells.

On the contrary, adult stem cells are not rejected by the immune system if obtained by autologous transplant. Furthermore, the fact that they are partially compromised reduces the number of differentiation stages necessary to generate specialised cells. In addition, the use of this type of cells is not associated with any type of legal or ethical controversy. Moreover, although these types of cells have less differentiation potentiality than ES cells, most of them are really multipotent (Joshi and Enver, 2002) which means that they can be differentiated to more than one type of tissue. What this suggests is that if an adequate source of adult stem cells is obtained, we could provide different cell types capable or covering multiple therapeutic applications.

However, an important disadvantage of using adult stem cells lies in their scarcity, which makes any process for obtaining and isolating this type of cell difficult and costly. An added problem is that most of the existing sources for obtaining stem cells are contaminated with other cell types, which complicates the process of identifying, isolating and characterising the stem cell population intended for therapeutic use or other uses.

At this time, the best sources of adult stem cells are: bone marrow (Spangrude et al., 1988, Osawa et al., 1996; Bhatia et al., 1997; Fridenshtein, 1982; Prockop, 1997; Pittenger et al., 1999; US Patent 5.486.359), peripheral blood (Barr and McBride, 1982; Russel and Hunter, 1994), umbilical cord (Broxmeyer et al., 1989), neural tissue (McKay, 1997); Johansson et al, 1999; Doetsch et al., 1999; Gage, 2000), adipose tissue (Zuk, et al., 2001; Zuk et al., 2002, patent application WO 03/022988), cornea (Daniels, et al., 2001); skin (Watt, 2001; Toma et al., 2001) gastrointestinal epithelium (Marshman et al., 2002) muscle (Grounds et al., 1992), liver (Forbes et al., 2003) and dental pulp (Gronthos et al., 2000; Miura et al., 2003). However, to date none of these sources has been capable of providing adult stem cells that meet each and every one of the following requirements: multipotentiality, reproducible tests, absence of contamination and perfect characterisation.

A new type of mammal stem cell called "Multipotent Adult Progenitor Cell (MAPC) was recently isolated from bone marrow and other tissues (Reyes et al., 2001; Jiang et al., 2002a; Jiang et al., 2002b; patent application WO 01/11011). This type of stem cells appears to be the progenitor of the so-called mesenchymal stem cells and shows a great deal of multipotentiality. However, the process of isolating and cultivating them is long and costly, and it includes the use of large quantities of diverse growth factors.

There is, therefore, a need to obtain an easily available source of multipotent stem cells. In particular, cells that can be easily isolated from a live subject without involving significant risk or pain, without high isolation and culturing costs and with minimal contamination from other cell types.

Cartilage is a tissue composed of a singular cellular element, chondrocytes, and an extracellular matrix (ECM) surrounding the chondrocytes. Thanks to its simple structure and cellular composition, cartilage could be a promising potential source of stem cells if these cells could be identified and characterised. Furthermore cartilaginous tissue can be extracted using minimally invasive procedures in comparison with other procedures (e.g., bone marrow extraction) and with low contamination compared to other procedures (e.g., extraction of adipose tissue), and without serious repercussions for the patient.

Adult articular cartilage is avascular, alymphatic, aneural and is nourished by synovial fluid (Mankin and Brandt, 1984). The only cells present in articular cartilage are chondrocytes, responsible for the synthesis, maintenance and renovation of ECM, which is in turn fundamentally composed of a network of highly hydrated collagen fibres inserted in a gel of charged proteoglycans (Maroudas, 1979). The digestion of ECM using collagenase allows the isolation the chondrocytes that can subsequently be grown and expanded *in vitro* (Mitrovic et al., 1979).

It is known that the single layer culturing of articular cartilage invariably leads to dedifferentiation, a process during which the cells recover their ability to divide, lose their rounded phenotype and stop producing collagen types II, IX and XI to produce types I, II and V (Mayne et al., 1976; von der Mark et al., 1997; Benya et al., 1977; Benya et al., 1978; Benya and Mimni, 1979; Benya and Shafter, 1982; Finer et al., 1985; Elima et al., 1989). Some authors have demonstrated that dedifferentiated chondrocytes of embryonic (Hegert et al., 2002) or adult (Tallheden et al., 2003) origin could be differentiated in vitro to several mesenchymal cell types, but thus far no one has isolated and characterised in detail a defined population of adult stem cells isolated from articular cartilage or demonstrated their multipotentiality.

The invention provides a population of multipotent adult stem cells from mammal cartilage, preferably from human articular cartilage, isolated and characterised in detail and also demonstrating their multipotentiality. These and other embodiments of the invention will be made apparent through the Description, Figures and Examples that follow.

### BRIEF DESCRIPTION OF THE INVENTION

A first aspect of the invention consists in providing an isolated population of multipotent stem cells derived from dedifferentiated chondrocytes perfectly characterised and free of other cell types. The chondrocytes are preferably obtained from human articular cartilage by arthroscopy, which is a routine medical procedure that involves minimal risk and discomfort for the patient.

A second aspect of the invention consists of obtaining, in vitro, from the said multipotent stem cells derived from dedifferentiated chondrocytes, cell populations differentiated to diverse lineages, including but not limited to the mesenchymal and neural lineages.

A third aspect of the invention consists of providing a transgenic cell population derived from the previously-mentioned isolated cells by modifying their genome.

A fourth aspect of the invention consists of using the previously-mentioned isolated stem cells for the preparation of pharmaceutical compositions that can be used for organ and tissue regeneration. The said pharmaceutical compounds constitute an additional aspect of this invention.

A fifth aspect of the invention consists of using the previously-mentioned isolated stem cells to evaluate the biological activity of different agents *in vitro* and *in vivo.*

Other aspects of this invention would be evident for an expert in the field in view of the description of the invention.

### BRIEF DESCRIPTION OF THE FIGURES

Figure 1 is a phase contrast microscopy picture of the stem cells of this invention.
Figure 2A shows fluorescent immunocytometry histograms corresponding to the positive surface markers on the stem cells of this invention. The histograms with black fill correspond to the marking with the specific antibody while the empty ones correspond to staining with the isotope control.
Figure 2B shows fluorescent immunocytometry histograms corresponding to the negative surface markers on the stem cells of this invention. The filled in area (black) corresponds to the histogram marked with the specific antibody; the blank area (white) corresponds to staining with the isotype control.
Figure 3A is a phase contrast microscopy picture of the stem cells of this invention differentiated in vitro to osseous phenotype. The differentiated cells were stained with Alizarin red to detect the calcium phosphate matrix secreted by the differentiated cells.
Figure 3B is a phase contrast microscopy picture of the stem cells of this invention without differentiation, stained by the same procedure as the differentiated cells in Figure 3A.
Figure 4A is a phase contrast microscopy picture of the stem cells of this invention differentiated in vitro to muscular phenotype. The differentiated cells were stained with a specific antibody for myosin heavy chain, a muscle-specific antigen.
Figure 4B shows a bright field microscopy of the stem cells of this invention without differentiation, stained by the same procedure as the differentiated cells in Figure 4A.
Figure 5A shows immunofluorescent microscopy of the stem cells of the invention differentiated in vitro to neuronal phenotype. The differentiated stem cells were stained with a specific antibody for NF200, a neuron-specific antigen.
Figure 5B shows immunofluorescent microscopy of the stem cells of the invention differentiated in vitro to neuronal phenotype. The differentiated stem cells were stained with a specific antibody for TuJ1, a neuron-specific antigen.
Figure 6 is a graphic representation of the number of clones isolated from the stem cells of this invention capable of differentiating to three different mesodermal tissues (AOC; adipose=A; osseous=O; cartilage=C) to two of them (AO, AC, OC), to one (A, O,C) or to none (-).
Figure 7A is an immunofluorescent photomicrograph of the stem cells of the invention transduced with a retroviral vector coding the green fluorescent protein (GFP).
Figure 7B is a fluorescent cytometry histogram that quantifies the fluorescence of the retrovirally transduced cells in Figure 7A.

### DETAILED DESCRIPTION OF THE INVENTION

First of all, this invention provides an isolated population of multipotent stem cells derived from dedifferentiated mammal chondrocytes, characterised in detail and free of other cell types. The isolated cell population to which the invention refers will preferably come from the cartilagenous tissue of a primate, preferably a human being. Normally, the cell to which the invention refers will come from human articular cartilage, in particular, from the cartilagenous tissue of the knee articulation. The stem cells and their derivatives in this invention could be used for different applications, some of which include: therapies based on autologous and alogenic transplant; development of disease models, development of trials to search for genes and to search for and develop drugs.

In a particular embodiment, the invention provides a multipotent adult stem cell from dedifferentiated mammal chondrocytes, characterised in that it is positive for the following surface antigens: CD9, CD13, CD29, CD44, CD49a, CD49b, CD49c, CD49e, CD54, CD55, CD58, CD59, CD90, CD95, CD105, CD106, CD166, HLA-1 and beta2-microglobulin.

In a preferred embodiment, the invention provides a multipotent adult stem cell from dedifferentiated mammal chondrocytes, characterised by the following phenotype: positive for markers CD9, CD13, CD29, CD44, CD49a, CD49b, CD49c, CD49e, CD54, CD55, CD58, CD59, CD90, CD95, CD105, CD106, CD166, HLA-1 and beta2-microglobulin; negative for markers CD10, CD11b, CD14, CD15, CD16, CD18, CD19, CD28, CD31, CD34, CD36, CD38, CD45, CD49d, CD50, CD51, CD56, CD61, CD62E, CD62L, CD62P, CD71, CD102, CD104, CD117, CD133, HLA-II.

Isolated cell populations composed of or including the said multipotent adult stem cells from dedifferentiated mammal chondrocytes are particular embodiments of the invention.

The isolated multipotent stem cell to which the invention refers is obtained from dedifferentiated adult chondrocytes isolated from cartilage biopsies performed on live subjects. In the preferred embodiment of the invention, the cartilaginous tissue is isolated from a human subject. In humans, the preferred source of cartilaginous tissue is the knee joint, the preferred method of obtaining the cartilage being a biopsy using arthroscopy on the edges of the femoral condyle. If the cells of the invention are to be transplanted in a human subject, it is preferable that the cartilaginous tissue be isolated from the subject to perform an autologous transplant.

The chondrocytes can be isolated from a cartilage biopsy using diverse methods known by experts in the field. Normally, enzymatic digestion with collegenase is used (Mitrovic et al., 1979). Example 1 of the invention details the procedure for isolating multipotent stem cells from dedifferentiated human chondrocytes obtained from knee articular cartilage.

The multipotent cells derived from the dedifferentiated chondrocytes can be characterised to identify the intracellular and/or surface proteins, genes and/or other markers indicative of their dedifferentiated status. Characterisation methods used include but are not limited to: immunocytometry (see Example 2), immunocytochemical analysis, northern blot analysis, RT-PCR, microarray gene expression analysis, proteomic studies and differential display analysis.

In another embodiment of the invention, the multipotent adult stem cells of the invention are induced to differentiate in vitro to cells that express at least one characteristic of a specialised cell. Such partial or totally differentiated cell types include but are not limited to cellular lineages characteristic of the following tissues and organs: cartilage, bone, fat, muscle, nerve tissue, skin, liver and pancreas, for example, chondrocytes, osteocytes, adipocytes, myocytes, carciomiocytes, neurons, astrocytes, oligodendrocytes, epithelial cells, hepatocytes, pancreatic cells, etc. The methods that can be used to induce the stem cells of the invention to differentiate to different types of specific cells are known by the experts in the field and some of them are explained in detail in the examples of the patent.

The partially or totally differentiated cells are characterised by the identification of surface and/or intracellular proteins, genes and other markers indicative of the differentiation of the stem cells of the invention to different lineages. Methods used for characterisation include but are not limited to: immunocytometry, immunocytochemical analysis, northern blot analysis, RT-PCR, analysis of genic expression in microchips, proteomic studies and differential display analysis.

In another aspect of the invention, the stem cells of the invention or cells derived therefrom are genetically modified either stably or transitorily to express exogenous genes or repress the expression of endogenous genes. Therefore, the invention provides an isolated transgenic cell population derived from the multipotent adult stem cells from the mammal-derived dedifferentiated chondrocytes provided by the invention whose genome has been modified by insertion of pre-selected isolated DNA, by the replacement of one segment of the cellular genome with pre-selected isolated DNA or by the inactivation of at least a portion of the cellular genome. According to this aspect of the invention, the isolated cells are put into contact with a gene transfer vector which contains a nucleic acid that includes a recombinant heterologous genetic sequence so that the nucleic acid is introduced in the cell under the appropriate conditions for the sequence to be expressed inside the cell. The gene transfer vector can be viral or non-viral. There are numerous viral and non-viral vectors for introduction exogenous DNA in the stem cells that are well known to experts in the field (Mulligan, 1993; Robbins et al., 1997; Bierhuizen et al., 1997). Viral vectors suitable for implementing this embodiment of the invention include but are not limited to the following: adenoviral vectors (Kozarsky and Wilson, 1993), adenoassociated vectors (Muzyczka, 1992), retroviral vectors (Tabin et al., 1982), lentiviral vectors (Naldini et al., 1996), alphaviral vectors (Huang, 1996), herpesviral vectors (Carpenter and Stevens, 1996) and coronavirus derived vectors (Orgego et al., 2002). Non-viral vectors suitable for implementing this embodiment of the invention include but are not limited to: raw DNA (Wolff et al., 1990), gen gun (Johnson et al., 1988), liposomes (Felgner et al., 1987), polyamines (Boussif et al., 1995), peptides (Wyman et al., 1997), dendrimer (Tang et al., 1996), cationic glycopolymers (Roche et al., 2003), liposome-polication compounds (Tsai et al., 1996), proteins (Fisher and Wilson, 1997) and receptor-mediated gene transfer systems (Cotton et al., 1990). The recombinant heterologous genetic sequence is normally included in an expression cassette that consists of a coded sequence operatively associated with a promoter or other cis sequences that permit their expression. The coding sequence may be used to code a protein, biologically active RNA, antisense RNA (Spampinato et al., 1992), a ribozime (Leavitt et al., 1994) or SiRNA (Qin et al., 2003). In a preferred embodiment, the stem cells of the invention are genetically modified to express a potentially therapeutic gene.

The stem cells of the invention, unmodified or genetically modified, and the cells derived therefrom that express at least one inherent characteristic of a specialised cell, either unmodified or genetically modified, can be used to prepare pharmaceutical compositions. In the preparation of said pharmaceutical components, the cells of the invention can be used alone or in conjunction with biologically compatible compositions, which may include but are not limited to: growth factors, cytokines, chemokines, extracellular matrix proteins, synthetic polymers and drugs. Therefore, in a particular embodiment of this invention, a pharmaceutical composition is provided which includes a population of stem cells provided by the invention, unmodified or genetically modified, or which express at least one inherent characteristic of a specialised cell, unmodified or genetically modified, and a pharmaceutically acceptable vehicle. In a special embodiment, said pharmaceutical component may also contain growth factors, cytokines, chemokines, extracellular matrix proteins, synthetic polymers and/or drugs. In a preferred embodiment of the invention, the shape of the pharmaceutical compositions prepared from the cells of the invention is that of a three-dimensional structure in which the cells and other possible components are included in a biocompatible three-dimensional synthetic matrix. Alternatively, said pharmaceutical compositions are of microparticule, microsphere, nanoparticle or nanosphere type structure.

The previously described implants are used in autologous and alogenic transplant procedures. These transplant procedures may be carried out administering the implants in a patient in different ways. The preferred forms of administration included but are not limited to: parenteral, intraperitoneal, intravenous, intradermal, epidural, intraspinal, intrastomal, intrarticular, intrasynovial, intratecal, intralesional, intraarterial, intracardiac, intramuscular, intranasal, intracraneal, subcutaneous, intraorbital, intracapsular, topical, by transdermal patches, rectally, vaginally, urethrally, by the administration of suppository, percutaneous, nasal spray, surgical implant, internal surgical pintura, infusion pump or by catheter.

The preferred therapeutic use of the cells described in the invention are intended to treat degenerative, traumatic, genetic, infectious or neoplasic diseases in humans resulting in damage to or dysfunction of organs or tissues that include but are not limited to: fistulas, ulcers, cartilage lesions, bone lesions, muscular lesions, muscular disorders (including but not limited to muscular dystrophy), bone diseases (including but not limited to imperfect osteogenesis), myocardial lesions, neurodegenerative disorders (including but not limited to: Parkinson's disease, Huntington's disease and Alzheimer's), spinal chord injuries, nerve damage, vascular lesions, skin lesions, liver damage and diabetes. Preferred embodiments of the genetically modified cells of the invention include but are not limited to: enzymatic substitution therapy, replacement of damaged cells and tissues, correction of deleterious genetic mutations, antiangiogenic therapy, prangiogenic therapy, anti-inflammatory therapy, release of bioactive compounds and release of anti-tumour agents.

Therefore, in a particular embodiment, the invention is related to the use of a population of stem cells provided by the invention, unmodified or genetically modified, or that express at least one inherent characteristic of a specialised cell, unmodified or genetically modified, to prepare a pharmaceutical composition for the treatment of lesions, degenerative and genetic diseases of cartilage, bones, muscles, the heart, central and peripheral nervous system, skin, liver and pancreas. By way of illustration, said isolated cell population provided by the invention can be used to prepare a pharmaceutical composition adequate for the treatment of cartilage lesions, or bone lesions, or muscular lesions, or cardiac lesions, or lesions of the peripheral nervous system, or lesions of the central nervous system, or skin lesions, or degenerative hepatic lesion, or degenerative pancreatic lesions, or for the treatment of genetic cartilage disorders, or genetic bone disorders, or genetic muscle tissue disorders, or genetic heart disorders, or genetic peripheral nervous system disorders, or genetic central nervous system disorders, or genetic skin disorders, or genetic hepatic disorders or genetic pancreatic disorders.

The presence of differentiated cells of the isolated multipotent stem cells of the invention on the subject on whom the transplant has been performed could be detected using diverse techniques which include but are not limited to: *in vivo* imaging, flow cytometry analysis, PCR analysis, southern blot analysis and immunohistochemcial studies.

In another aspect of the invention, the stem cells of this invention, with or without genetic modification, as well as the cells derived therefrom that express at least one inherent characteristic of a specialised cell, with or without genetic modification, can be applied to the development of in vitro and in vivo tests for the following industrial purposes: search for drugs, pharmacological studies, toxicological studies, pharmacogenomic studies and genetic studies. Such studies can be used to identify and/or characterise a multitude of biological targets, bioactive compounds or pharmacological agents.

The stem cells of this invention provide a unique system in which the cells can be differentiated to give rise to specific lineages of the same individual. In addition, the cells of this invention provide a source of cells in culture from a potential variety of genetically diverse individuals that can respond differently to different biological and pharmacological agents. When comparing the responses of the cells from a statistically significant population of individuals, it is possible to determine the effects of the biological or pharmacological agents being tested on a specific type of cell. Unlike most primary cultures, the cells of this invention can be maintained in culture and hence can be studied over time. Therefore, multiple cell cultures from the same or different individuals can be treated with the agent of interest to determine if there are differences in the effects of that agent on certain types of cells with the same genetic profile or, alternatively, on similar cell types from genetically different individuals.

The use of the stem cells of this invention in a high-throughput screening system makes it possible to analyse a wide range of biological and pharmaceutical agents and combinatorial libraries of same, effectively in terms of time and money, so as to clarify their effects on human cells. These agents include but are not limited to: peptides, antibodies, cytokines, chemokines, growth factors, hormones, viral particles, antibiotics, inhibitor compounds, chemotherapy agents, cytotoxic agents, mutagens, food additives, pharmaceutical compositions and vaccines.

In the pharmacogenomics field, the stem cells of the invention isolated from a statistically significant population of individuals can be used to provide an ideal system for identifying polymorphisms associated with positive or negative responses to a wide range of substances. The information obtained from these studies can have significant repercussions on the treatment of infectious diseases, cancer and diverse metabolic disorders.

The in vitro method that allows the stem cells of the invention to be used to evaluate the cellular response to biological or pharmacological agents or to combinatorial libraries of those agents, includes the following:
a) isolating the cells provided by the invention from an individual or statistically significant population of same;
b) optionally differentiating the isolated cells to a specific cell type;
c) expanding the cells in culture;
d) optionally differentiating the isolated cells expanded to a specific cell type;
e) putting the culture in contact with one or more biological or pharmacological agents or with a combinatorial library of those agents and
f) evaluating the possible biological effects of those agents on the cultured cells.

Alternatively, in order to implement this method, the stem cells provided by this invention can be used, optionally genetically modified or cells that express at least one inherent characteristics of a specialised cell, optionally genetically modified.

In the previously described method, the biological or pharmacological agents that can be evaluated include but are not limited to peptides, antibodies, cytokines, chemokines, growth factors, viral particles, hormones, drugs, for example, antibodies, chemotherapy agents, cytotoxic agents, pharmaceutical compounds, vaccines, extracellular matrix proteins, synthetic polymers, inhibitor compounds, mutagens, food additives, etc.

The in vivo method that allows the stem cells of the invention to be used to evaluate the cellular response to biological or pharmacological agents or to combinatorial libraries of those agents, includes the following:
a) isolating the cells provided by the invention from an individual or statistically significant population of same;
b) optionally differentiating the isolated cells to a specific cell type;
c) expanding the cells in culture;
d) optionally differentiating the isolated cells expanded to a specific cell type;
e) implanting the cells, alone or within biologically compatible compositions, in an experimental animal model;
f) administering one or more biological or pharmacological agents to the grafted animals;
g) evaluating the possible biological effects of those agents on the implanted cells.

Alternatively, in order to implement this method, the stem cells provided by this invention can be used, optionally genetically modified or cells that express at least one inherent characteristic of a specialised cell, optionally genetically modified.

In the previously described method, the experimental animal may be, but is not limited to, a strain of an immunodeficient mouse. In this method, the biologically compatible compositions may included but are not limited to: peptides, antibodies, cytokines, chemokines, growth factors, viral particles, hormones, drugs, for example, antibiotics, chemotherapy agents, cytotoxic agents, pharmaceutical compounds, vaccines, extracellular matrix proteins, synthetic polymers, inhibitor compounds, mutagens, food additives, etc. In a preferred embodiment of the invention, the cells are implanted in an experimental animal contained in a biocompatible three-dimensional synthetic matrix. In another embodiment of the invention, the cells are introduced in the animal contained in a microparticle, microsphere, nanoparticle or nanosphere type structure. The preferred forms of implanting the said cells, compositions and structures in the experimental animal include but are not limited to: parenteral, intraperitoneal, intravenous, intradermal, epidural, intraspinal, intrastomal, intrarticular, intrasynovial, intratecal, intralesional, intraarterial, intracardiac, intramuscular, intranasal, intracraneal, subcutaneous, intraorbital, intracapsular, topical, by transdermal patches, rectally, vaginally, urethrally, by the administration of suppository, percutaneous, nasal spray, surgical implant, internal surgical pintura, infusion pump or by catheter.

Certain embodiments of the invention are described in more detail below.

### EXAMPLES

The following are given to illustrate but do not limit the invention.

### Example 1: Isolation of chondrocyte-derived multipotent stem cells obtained from human articular cartilage.

The procedure begins by obtaining a biopsy of cartilage from the outside edges of the femoral condyle by arthroscope. The size of the biopsy may vary depending on the structure of the articulation, the patient's age and the surgeon's discretion, but it is normally not smaller than 4 cm². The biopsy is placed in a sterile saline solution at 4°C until processing, which should not take place more than 48 hours after the sample is taken.

The cartilage biopsy is suspended in 1 millilitre of sterile basal culture medium (DMEM, Dulbecco Modified Eagle's Medium) containing L-glutamine 2 mM, antibiotics and 1% bovine fetal serum (BFS). The serum may also be of human origin, preferably autologous. The cartilage is then ground up using surgical scissors under aseptic conditions. The resulting cartilage fragments are added to a suspension containing 0.1% collagenase in the same medium used for the grinding, and the resulting cellular suspension is incubated for at least 4 hours at 37°C, agitating gently. The resulting cellular suspension is then filtered through a 40 micrometer sterile mesh and then the filtered suspension is centrifuged at 500 g for 5 minutes. The resulting cellular sediment is then resuspended in a culture medium and cultured at a density of approximately 20,000 cells/cm³ in tissue culture flasks. The culture medium is normally composed of a basal medium such as DMEM, L-glutamine 2 mM, 10% BFS and antibiotics (Choi et al., 1980; Webber and Scokoloff, 1981). Another possibility is to use human serum from an autologous source rather than bovine serum. Another possibility is to use a defined culture medium that contains a basal medium like DMEM, RPMI, F12 or a combination of these, L-glutamine 2mM, antibiotics and a supplementary medium that includes but is not limited to the following: insulin, transferrin, selenium, albumina, and linoleic acid (Kato et al., 1980; Schwartz and Sugumaran, 1982; Jennings et al., 1983; Adolphe et al., 1984; Quarter et al., 1997, US patent 6.150.163).

The cells are subsequently cultivated in an incubator at 37°C with 5% CO₂ and 95% humidity. After four days of culturing, the medium is removed, the non-adhered cells are eliminated using a saline phosphate tampon (PBS) and fresh medium is added. After another four days, the cells are rinsed again and detached by incubating them with a solution containing 0.25% trypsin and 0.02% EDTA (ethylenediaminetetraacetic acid). The detached cells are centrifuged to sediment them, counted, and seeded at a density of 5,000 cells/cm2 in new culture flasks. The cells are maintained in a single layer in culture in a state of subconfluence by detachment and re-seeding at 5,000 cells/cm². The resulting adhered cells are isolated multipotent stem cells that can be maintained dedifferentiated in the culture conditions described above. Figure 1 shows a photomicrograph of said cells after 15 days in culture.

### Example 2. Immunophenotypical characterisation of multipotent stem cells derived from dedifferentiated human chondrocytes.

The multipotent stem cells from dedifferentiated chondrocytes are collected by gentle digestion with trypsin, rinsed with PBS and incubated for 30 minutes at 4°C with one of the following antibodies labelled with FITC or PE: CD9, CD10, CD11b. CD13, CD14, CD15, CD16, CD18, CD19, CD28, CD29, CD31, CD34, CD36, CD38, CD44, CD45, CD49a, CD49b, CD49c, CD49e, CD50, CD51, CD54, CD55, CD56, CD58, CD59, CD61, CD62E, CD62L, CD62P, CD71, CD90, CD95, CD102, CD104, CD105, CD106, CD117, CD133, CD166, HLA-1, HLA-II and beta2-microglobulin.

The marked cells are rinsed and analysed immediately using an Epics-XL (Coulter) cytometre. The cells stained with unspecific antibodies of the isotypes marked with fluorescence (FITC) or phycoerythrin (PE) were used as controls. Figure 2A shows the histograms indicating the positive marking of the cells, while Figure 2B shows the histograms that indicate the absence of the corresponding antigen.

### Example 3: In vitro differentiation of multipotent stem cells derived from dedifferentiated human chondrocytes to osseous phenotype cells

The stem cells derived from dedifferentiated chondrocytes are seeded at a density of 20,000 cells/cm² in a standard culture medium (DMEM, 10%BFS, L-glutamine 2 mM and antibiotic). After 12 hours the culture medium is replaced by an osteogenesis inductor medium (Jaiswal et al., 1997) composed of:
- MEM
- 20% BFS
- Penicillin / streptomycin
- L-glutamine 2mM
- Dexamethasone 0.01µM
- Ascorbic acid 0.2 mM
- β-Glycerophosphate 10mM

Mineralised calcium phosphate deposits can be observed after 14 days which indicated the presence of bone nodules. The nodules are detected by staining with Alizarin Red (Standford et al., 1995) as detailed below: the medium is eliminated and rinsed with PBS; the samples are fixed with 70% ethanol for 1 hour at 4°C; the sample is stained with 1 ml Alizarin red 40 mM pH 4.1 and the colouring agent is eliminated after 5 minutes with abundant water. Figure 3A shows the results obtained after staining the differentiated cells, while Figure 3B shows the results of the staining of the undifferentiated cells.

### Example 4: In vitro differentiation of multipotent stem cells derived from dedifferentiated human chondrocytes to muscular phenotype cells

The stem cells derived from dedifferentiated chondrocytes are seeded at a density of 10,000 cells/cm² in a standard culture medium (DMEM, 10% BFS, L-glutamine 2 mM and antibiotic). After 12 hours the culture medium is replaced by a miogenesis inductor medium (Wakitani et al., 1995) composed of:
- DMEM
- 20% BFS
- Penicillin / streptomycin
- L-glutamine 2mM
- Ascorbate-2-phosphate 0.1 mM
- Dexamethasone 0.01µM
- ITS+1 (Sigma-Aldrich)
- 5-Azacitidine 3 µM

Mineralised calcium phosphate deposits can be observed after 14 days which indicated the presence of osseous nodules. The nodules are detected by staining with Alizarin Red (Standford et al., 1995) as detailed below: the medium is eliminated and rinsed with PBS; the samples are fixed with 70% ethanol for 1 hour at 4°C; the sample is stained with 1 ml Alizarin red 40 mM pH 4.1, and the colouring agent is eliminated after 5 minutes with abundant water. Figure 3A shows the results obtained after staining the differentiated cells, while Figure 3B shows the results of the staining of the undifferentiated cells.

24 hours later the medium is replaced with a standard culture medium and the cells are cultured for 2-3 weeks, changing the medium twice a week. After that, the cells acquire an elongated phenotype, form fibrilar structures and some cellular fusions can be observed. To detect the myoblast phenotype, the cells obtained are fixed with 4% paraformaldehyede (PFA) and incubated with an antibody for the myosin heavy chain, a muscle-specific antigen. Figure 4A shows the results obtained after the staining of the differentiated cells, while Figure 4B shows the results of the staining of the undifferentiated cells.

### Example 5: In vitro differentiation of multipotent stem cells derived from dedifferentiated human chondrocytes to neuronal phenotype cells

The stem cells derived from dedifferentiated chondrocytes are seeded at low density in a standard culture medium (DMEM, 10% BFS, L-glutamine 2 mM and antibiotic), supplemented with 10 ng/ml Bfgf and incubated for 24-36 hours to achieve a high cellular confluence. The cells are then rinsed and a neuroinductor medium is added (Black and Woodbury, 2001) composed of:
- MEM
- BHA 200 µM
- Penicillin / streptomycin
- L-glutamine 2mM
- Phaskoline 10 µM
- 2% DMSO
- Hydrocortisone 1µM
- Insulin 5 µg/ml
- CIK 25 mM
- Valproic acid 2mM

Within a few hours of the induction a morphological change can be observed in which the cells acquire a rounded and very refringent cellular body and prolongations similar to the axons and dendrites of nerve cells. After 3 days, the cells obtained are fixed with 4% PFA and incubated with antibodies to the neuron-specific antigens NF-200 and TuJ1. Using this procedure, it is observed that 30% of the cells are positive for NF-200 (Figure 5A) and 75% of the cells are positive for TuJI (Figure 5B).

### Example 6: Demonstration of the clonal multipotentiality of stem cells derived from dedifferentiated human chondrocytes.

The stem cells derived from dedifferentiated chondrocytes are seeded on 96-well dishes at a rate of one cell per well applying the limit dilution method in a standard culture medium (DMEM, 10% FSB, L-glutamine 2mM and antibiotic). After 2 hours, the presence of a single cell in each well is confirmed on microscopic observation and the wells containing more than one cell or no cells are discarded. The cultures are allowed to evolve until high cellular confluence is achieved, changing the medium twice a week. The clones are subcultured on a larger surface as they achieve higher cellular confluence. The cloning efficiency is between 50-60%. Morphological differences are not seen between the different clones obtained. Once the clones are expanded, osteogenic, adipogenic and chondrogenic differentiation procedures are carried out as follows:
1. Osteogenic differentiation. Carried out as described in Example 3.
2. Adipogenic differentiation. The stem cells derived from dedifferentiated chondrocytes are seeded at a density of 20,000 cells/cm2 in a standard culture medium (DMEM, 10% BFS, L-glutamine 2mM and antibiotic). 12 hours later, the adipogenesis inducer is added to the medium (Pittenger et al., 1999) composed of:
   - MEM
   - 20% BFS
   - Penicillin / streptomycin
   - L-glutamine 2mM
   - Hydrocortisone 0.5 µM
   - IBMX 0.5 µM
   - Indomethacine 60 µM

   24 days after differentiation, it can be observed that there are cytoplasmic lipidic vacuoles characteristic of adipose cells. Said vacuoles are detected by a staining with Red Oil (Ramirez-Zacarias et al., 1992) as described below: after eliminating the medium and rinsing with PBS, the samples are fixed with formalin from 30 to 60 minutes at room temperature; they are rinsed with water; they are incubated with 60% isopropanol for 3 minutes,; the isopropanol is eliminated and a solution of Red Oil is added and left for 5 minutes, after which it is eliminated by rinsing with abundant water.
3. Chondrogenic differentiation. Start with 5 x 10⁵ cells that are sedimented by centrifugation at 400 g for 5 minutes in a polypropylene conic tube. They are then incubated in 2 ml of standard culture medium (DMEM, 10% bovine fetal serum, L-glutamine 2 mM and antibiotic) and 24 hours later it can be seen that a compact spherical structure has formed that is no longer adhered to the base of the tube. The cells continue to culture, changing the culture medium twice a week. Two weeks later, after rinsing with PBA, the cellular aggregates are fixed with a solution of 4% paraformaldehyde for 90 minutes at room temperature. They are then included in paraffin. The blocks with the samples included are cut at a thickness of 4 µm with a microtome. The presence of proteoglicans characteristic of this type of tissue is revealed by staining with Alcian Blue (Lev et al., 1964) as described below: the samples are deparaffined and hydrated and stained with a solution of Alcian Blue prepared in hydrochloric acid 0.1 N for 30 minutes; they are then dehydrated and assembled with a resinous medium. As a result of the process one can see the stained blue sulphated proteoglycans. Immunofluorescence against the type II collagen molecule was also conducted, which is expressed by the chondrocytic cells being one of the principal components of extracellular matrix of cartilage.

The results of the experiment show that a very high percentage (33%) of the clones obtained are multipotent (see Figure 6).

### Example 7. Expression by retroviral transduction of a heterological gene in multipotent stem cells derived from dedifferentiation human chondrocytes.

The stem cells derived from dedifferentiated chondrocytes are plated at 15,000 cells /cm² and incubated at 37°C for 6 hours with a preparation of retroviral particles coding the green fluorescent protein (GFP) and packaged with an amphotropic envelope. After infection, the cells are rinsed with a phosphate tampon and kept in the habitual culture medium. After 48 hours, the expression of GFP can be analysed using fluorescence microscopy (see Figure 7A) or using flow cytometry (see Figure 7B).

**REFERENCES CITED PATENTS**

| | |
|---|---|
| US 6.150.163 | McPherson *et al.* |
| US 5.811.094 | Binette *et al.* |
| US 5.958.767 | Kim *et al.* |
| US 6.328.960 | Klyushnenkova *et al.* |
| US 6.379.953 | Bruder *et al..* |
| US 6.497.875 | Sorrell *et al.* |
| US 6.294.346 | Reinolds *et al.* |
| US 6.200.806 | Thomson *et al.* |
| US 5.486.359 | Haynesworth *et al.* |
| WO 03/022988 | Futrell *et al.* |
| WO 01/11011 | Reyes *et al.* |

### OTHER DOCUMENTS

Adolphe, M. *et al.* (1984) Exp Cell Res 155: 527-536.
Barr, R.D., McBride, J.A. (1982) Br J Haematol 51: 181-187.
Benya, P.D., Nimmi, M.E. (1979) Arch Biochem Biophys 192: 327-335.
Benya, P.D. *et al.* (1977) Biochemistry 16: 865-872.
Benya, P.D. *et al.* (1978) Cell 15: 1313-1321.
Benya, P.D., Shaffer, J.D. (1982) Cell 30: 215-224.
Bhatia, M. *et al.* (1997) Proc Natl Acad Sci U S A 94: 5320-5325.
Bierhuizen, M.F. *et al.* (1997) Biochem Biophys Res Commun 234: 371-375.
Black, LB., Woodbury, D. (2001) Blood Cells Mol Dis 27(3): 632-636.
Boussif, O*. et al.* (1995) Proc Natl Acad Sci U S A 92: 7297-7301.
Broxmeyer, H.E*. et al.* (1989) Proc Natl Acad Sci U S A 86: 3828-3832.
Carpenter, D.E., Stevens, J.G. (1996) Hum Gene Ther 7: 1447-1454.
Choi, Y.C. *et al.* (1980) Connect Tissue Res 7: 105-112.
Cotten, M*. et al.* (1990) Proc Natl Acad Sci U S A 87: 4033-4037.
Daniels, J.T*. et al.* (2001) Wound Repair Regen 9: 483-494.
Doetsch, F. *et al.* (1999) Cell 97: 703-716.
Elima, K., Vuorio, E. (1989) FEBS Lett 258: 195-198.
Evans, M.J., Kaufman, M.H. (1981) Nature 292: 154-156.
Evans *et al.* (1992) J Am Med Assoc 267: 239-246.
Felgner, P.L. *et al.* (1987) Proc Natl Acad Sci U S A 84: 7413-7417.
Finer, M.H. *et al.* (1985) Mol Cell Biol 5: 1415-1424.
Fisher, K.J., Wilson, J.M. (1997) Biochem J 321 (Pt1): 49-58.
Forbes, S. *et al.* (2002) J Pathol 197: 510-518.
Fridenshtein, A. (1982) Arkh Patol 44: 3-11.
Fuchs, E., Segre, J.A. (2000) Cell 100:143-155.
Gage, F.H. (2000) Science 287: 1433-1438.
Gronthos, S. *et al.* (2000) Proc Natl Acad Sci U S A 97: 13625-13630.
Grounds, M.D*. et al.* (1992) Cell Tissue Res 267: 99-104.
Hegert, C*. et al.* (2002) J Cell Sci 115: 4617-4628.
Huang, H.V. (1996) Curr Opin Biotechnol 7: 531-535.
Ivanova, N.B. *et al.* (2002) Science 298: 601-604.
Jaiswal, N*., et al.* (1997) J Cell Biochem 64: 295-312.
Jayawickreme, C.K., Kost, T.A. (1997) Curr Opin Biotechnol 8: 629-634.
Jennings, S.D., Ham, R.G. (1983) Cell Biol Int Rep 7: 149-159.
Jiang, Y. *et al.* (2002) Nature 418: 41-49.
Jiang, Y*. et al.* (2002) Exp Hematol 30: 896-904.
Johansson, C.B. *et al.* (1999) Cell 96: 25-34.
Johnston, S.A. *et al.* (1988) Science 240: 1538-1541.
Joshi, C.V., Enver, T. (2002) Curr Opin Cell Biol 14: 749-755.
Kato, Y *et al.* (1980) Exp Cell Res 125: 167-174.
Kozarsky, K.F., Wilson, J.M. (1993) Curr Opin Genet Dev 3: 499-503.
Leavitt, M.C. *et al.* (1994) Hum Gene Ther 5: 1115-1120.
Lev, R*., et al.* (1964) J Histochem Cytochem 12:309.
Mankin, H.J., Brandt, K.D. (1984) "Osteoarthritis" ed. Moskowitz, et al., WB Saunders, Philadelphia, pp. 43-79.
Maroudas, N.G. (1979) J Theor Biol 79: 101-116.
Marshman, E. *et al.* (2002) Bioessays 24: 91-98.
Mayne, R*. et al.* (1976) Proc Natl Acad Sci U S A 73: 1674-1678.
McKay, R. (1997) Science 276: 66-71.
Mitrovic, D*. et al.* (1979) J Rheumatol 6: 124-130.
Miura, M*. et al.* (2003) Proc Natl Acad Sci U S A 100: 5807-5812.
Mulligan, R.C. (1993) Science 260: 926-932.
Muzyczka, N. (1992) Curr Top Microbiol Immunol 158: 97-129.
Naldini, L. *et al.* (1996) Science 272: 263-267.
Ortego, J. *et al.* (2002) J Virol 76: 11518-11529.
Osawa, M. *et al.* (1996) Science 273: 242-245.
Patience, C. *et al.* (1997) Nat Med 3: 282-286.
Phillips, R.L. *et al.* (2000) Science 288: 1635-1640.
Pittenger, M.F. *et al.* (1999) Science 284: 143-147.
Prockop, D.J. (1997) Science 276: 71-74.
Qin, X.F. *et al.* (2003) Proc Natl Acad Sci U S A 100: 183-188.
Quarto, R. *et al.* (1995) Calcif Tissue Int 56: 123-129.
Ramalho-Santos, M. *et al.* (2002) Science 298: 597-600.
Ramirez-Zacarias, J.L. *et al.* (1992) Histochemistry 97: 493-497.
Reyes, M., Verfaillie, C.M. (2001) Ann N Y Acad Sci 938: 231-233; discussion 233-235.
Robbins, P.B. *et al.* (1997) J Virol 71: 9466-9474.
Roche, A.C. *et al.* (2003) Cell Mol Life Sci 60: 288-297.
Russell, N.H., Hunter, A.E. (1994) Bone Marrow Transplant 13: 353-355.
Schwartz, E.R., Sugumaran, G. (1982) In Vitro 18: 254-260.
Spampinato, S. *et al.* (1992) Pharmacol Res 25 Suppl 1: 51-52.
Spangrude, G.J. *et al.* (1988) Science 241: 58-62.
Stanford, C.M. *et al.* (1995) J Biol Chem 270: 9420-9428.
Tabin, C.J. *et al.* (1982) Mol Cell Biol 2: 426-436.
Tallheden, T. *et al.* (2003) J Bone Joint Surg Am 85-A Suppl 2: 93-100.
Tang, M.X. *et al.* (1996) Bioconjug Chem 7: 703-714.
Thomson, J.A*. et al.* (1998) Science 282: 1145-1147.
Toma, J.G*. et al.* (2001) Nat Cell Biol 3: 778:784.
Tsai, J.T. *et al.* (2002) Biotechnol Appl Biochem 36: 13-20. Von der Mark, K*. et al.* (1977) Nature 267: 531-532.
Wakitani S. *et al.* (1995) Muscle Nerve 18: 1417-1426.
Watt, F.M. (2001) Curr Opin Genet Dev 11: 410-417.
Webber, R.J., Sokoloff, L. (1981) Growth 45: 252-268.
Wilson, C.A*. et al.* (1998) J Virol 72: 3082-3087.
Wolff, J.A. *et al.* (1990) Science 247: 1465-1468.
Wyman, T.B. *et al.* (1997) Biochemistry 36: 3008-3017.
Zuk, P.A*. et al.* (2002) Mol Biol Cell 13: 4279-4295.
Zuk, P.A. *et al.* (2001) Tissue Eng 7: 211-228.

## Claims

1. An isolated population of adult multipotent stem cells from dedifferentiated chondrocytes of mammal articular cartilage **characterised in that** they are positive for the following surface antigens: CD9, CD13, CD29, CD44, CD49a, CD49b, CD49c, CD49e, CD54, CD55, CD58, CD59, CD90, CD95, CD105, CD106, CD166, HLA-1 and beta2-microglobulin.

2. An isolated population of adult multipotent stem cells according to claim 1, **characterised in that** they are negative for the following surface antigens: CD10, CD 11b, CD14, CD15, CD16, CD18, CD19, CD28, CD31, CD34, CD36, CD38, CD45, CD49d, CD50, CD51, CD56, CD61, CD62E, CD62L, CD62P, CD71, CD102, CD104, CD117, CD133, HLA-II.

3. An isolated population of adult multipotent stem cells according to claim 1, **characterised in that** the cells are of human origin.

4. An isolated cell population derived from an isolated population of adult multipotent stem cells according to any of claims 1 through 3, **characterised in that** it expresses at least one characteristic of a specialised cell.

5. An isolated cell population according to claim 4, **characterised in that** it expresses at least one characteristic of a chondrocyte.

6. An isolated cell population according to claim 4, **characterised in that** it expresses at least one characteristic of an osteocyte.

7. An isolated cell population according to claim 4, **characterised in that** it expresses at least one characteristic of an adipocyte.

8. An isolated cell population according to claim 4, **characterised in that** it expresses at least one characteristic of a myocyte.

9. An isolated cell population according to claim 4, **characterised in that** it expresses at least one characteristic of a cardiomyocyte.

10. An isolated cell population according to claim 4, **characterised in that** it expresses at least one characteristic of a neuron.

11. An isolated cell population according to claim 4, **characterised in that** it expresses at least one characteristic of an astrocyte.

12. An isolated cell population according to claim 4, **characterised in that** it expresses at least one characteristic of an oligodendrocyte.

13. An isolated cell population according to claim 4, **characterised in that** it expresses at least one characteristic of an epithelial cell.

14. An isolated cell population according to claim 4, **characterised in that** it expresses at least one characteristic of a hepatocyte.

15. An isolated cell population according to claim 4, **characterised in that** it expresses at least one characteristic of a pancreatic cell.

16. An isolated transgenic cell population derived from the isolated cell population according to any of claims 1 through 15, **characterised in that** its genome has been modified by the insertion of pre-selected isolated DNA, by replacing a segment of the cellular genome with pre-selected isolated DNA or by inactivation of at least one portion of the cellular genome.

17. An isolated transgenic cell population according to claim 16 **characterised in that** its genome has been modified by non-viral transduction.

18. An isolated transgenic cell population according to claim 16 **characterised in that** its genome has been modified by viral transduction.

19. Use of an isolated cell population according to any of claims 1 through 18 to prepare a pharmaceutical composition for the treatment of lesions, degenerative and genetic diseases of: cartilage, bone, muscle, heart, central and peripheral nervous system, skin, liver and pancreas.

20. A pharmaceutical composition that includes a cell population according to any of claims 1 through 18 and an acceptable pharmaceutical vehicle.

21. A pharmaceutical compound according to claim 20 which also includes an additional component selected among growth factors, cytokines, chemokines, extracellular matrix proteins, drugs, synthetic polymers and mixtures.

22. A pharmaceutical composition according to claim 20 or 21 wherein the cells and, optionally, the additional components, are included in a three-dimensional biocompatible synthetic matrix.

23. A pharmaceutical composition according to claim 22 wherein said three-dimensional biocompatible synthetic structure is of a microparticle, microsphere, nanoparticle or nanosphere type.

24. Method for the in vitro evaluation of the cellular response to biological or pharmacological agents or to the combinatorial libraries of such agents, which includes:
a) isolating a cell population according to any of claims 1 through 18 from an individual or statistically significant population of same;
b) optionally differentiating the isolated cells to a specific cell type;
c) expanding the cells in culture;
d) optionally differentiating the isolated cells expanded to a specific cell type;
e) putting the culture in contact with one or more biological or pharmacological agents or with a combinatorial library of those agents and
f) evaluating the possible biological effects of those agents on the cultured cells.

25. Method according to claim 24 wherein said biological or pharmacological agents to evaluate include peptides, antibodies, cytokines, chemokines, growth factors, hormones, viral particles, antibiotics, inhibitor compounds, chemotherapy agents, cytotoxic agents, mutagens, food additives, pharmaceutical compositions and vaccines.

26. Method for the in vivo evaluation of the cellular response to biological or pharmacological agents or to the combinatorial libraries of such agents, which includes
a) isolating a cell population according to any of claims 1 through 18 from an individual or statistically significant population of same;
b) optionally differentiating the isolated cells to a specific cell type;
c) expanding the cells in culture;
d) optionally differentiating the isolated cells expanded to a specific cell type;
e) implanting the cells, alone or within biologically compatible compositions, in an experimental animal model;
f) administering one or more biological or pharmacological agents to the grafted animals;
g) evaluating the possible biological effects of those agents on the implanted cells.

27. Method according to claim 26 wherein the experimental animal used is an immunodeficient mouse strain.

28. Method according to claim 26 wherein the cells are implanted in the experimental animal inside a three-dimensional biocompatible matrix.

29. Method according to claim 26 wherein the cells are implanted in the experimental animal inside a microparticle, microsphere, nanoparticle or nanosphere type structure.

30. Method according to claim 26 wherein the biological or pharmacological agents to evaluate include peptides, antibodies, cytokines, chemokines, growth factors, hormones, viral particles, antibiotics, inhibitor compounds, chemotherapy agents, cytotoxic agents, mutagens, food additives, pharmaceutical compositions and vaccines.
